# EUROPÄISCHE PATENTSCHRIFT

(11) **EP 1 905 363 B1**
(45) Veröffentlichungstag und Bekanntmachung des Hinweises auf die Patenterteilung: **20.01.2010**
(21) Anmeldenummer: 07016318.3
(22) Anmeldetag: 21.08.2007
(51) Int. Cl.: A61B 17/16

(54) **Verfahren zum Bestimmen einer Zahnperiodenlänge eines Knochen-Fräsers**
Method for determining the tooth period length of a bone milling cutter
Procédé de détermination de la période spatiale des dents d'une fraise à os

(30) Priorität: 27.09.2006 DE 102006045508
(43) Veröffentlichungstag der Anmeldung: 02.04.2008
(73) Patentinhaber: joimax GmbH, 76227 Karlsruhe (DE)
(72) Erfinder: Ries, Wolfgang, 76351 Linkenheim (DE); Notheis, Mathias, 63517 Rodenbach (DE)
(74) Vertreter: Lempert, Jost

(56) Entgegenhaltungen:
- EP-A- 0 296 986
- EP-A- 1 609 560
- DE-T2- 69 917 683
- US-A- 5 759 185

## Beschreibung

Die Erfindung betrifft ein Verfahren und eine Vorrichtung zum Herstellen von Knochen-Fräsern, wobei an einer distalen Stirnseite eines rohrartigen Fräser-Schafts eines Knochen-Fräsers bei vorgegebenem Außendurchmesser des Fräser-Schafts, eine Fräser-Zahnung geschaffen wird.

Beispielsweise aus der DE 699 17 683 T2 ist ein Knochen-Fräser bekannt, der einen hohen zylindrischen Fräser-Schaft, an seinem rückwärtigen, proximalen Ende einen Handgriff und an seinem stirnseitigen bzw. distalen Ende eine Fräser-Zähnung aufweist.

Ein derartiger Fräser wird im Bereich der Medizintechnik zum Ausfräsen von Wirbelbestandteilen im Bereich eines seitlichen Fortsatzes eines Wirbelsäulen-Wirbels eingesetzt, um einen postero-lateralen Zugang zu eingeklemmten Nervenwurzeln des zentralen Nervensystems zu schaffen. Durch diesen Zugang werden dann Nucleus-Propulsus-Gewebe und andere Gewebearten (Kapselgewebe, Narbengewebe, Annulus-Gewebe) entfernt, da diese auf die Nervenwurzeln drükken. Der benannte Fortsatz eines Wirbels bildet mit einem benachbarten Fortsatz eines benachbarten Wirbels das so genannte Facettengelenk.

Die einen gattungsgemäßen Facettengelenkfräser einsetzende microinvasive Operationsmethode zur Dekompression von eingeklemmten Nervenwurzeln ist höchst erfolgreich. Wegen der hohen Sensibilität eines solchen Eingriffs müssen gattungsgemäße Knochen-Fräser hochgradig präzise gefertigt werden und eine hohe Festigkeit haben, um ein Abstumpfen der Zahnung zu vermeiden. Ferner muss ein solcher Knochen-Fräser im Gebrauch stabil sein und darf unter keinen Umständen abrutschen. All diese Anforderungen sind bei gleichzeitig einfacher und guter Desinfizierbarkeit zu erfüllen. Daher ist die Herstellung von Knochen-Fräsern der gattungsgemäßen Art, die zudem in kleinen Stückzahlen erfolgt, sehr teuer. Dies fällt insbesondere deshalb ins Gewicht, weil ein Chirurg im Allgemeinen nicht nur einen Knochen-Fräser benötigt sondern ein ganzes Sortiment mit verschiedenen Bemaßungen und Zahnungsmustern.

Ausgehend von diesem Stand der Technik liegt der Erfindung die Aufgabe zugrunde, unter vermeidung der genannten Nachteile ein Verfahren zum Herstellen einen Knochen-Fräsers, bei dem der Fertigungsaufwand gegenüber nach bekannten Verfahren bemessenen Fräser-Zähnungen verringert wird. Durch das Verfahren soll ein Fräser zudem derart bemessen werden können, dass für den Gebrauch des Knochen-Fräsers eine gute Stabilität und Festigkeit der Zahnung sichergestellt ist.

Erfindungsgemäß wird die genannte Aufgabe mit einem Verfahren der gattungsgemäßen Art gelöst, dass die Schritte des Anspruchs 1 aufweist.

Das erfindungsgemäße Verfahren kann besonders vorteilhaft in einer erfindungsgemäß ausgebildeten Vorrichtung zum Herstellen eines Knochen-Fräsers mit den kennzeichnenden Merkmalen des Anspruchs 18 implementiert sein, in welcher die Bemaßung von individuell oder in einer kleinen Serie gefertigten Knochen-Fräsern mit Hilfe des erfindungsgemäßen Verfahrens automatisch abhängig von wenigen Kenngrößen, beispielsweise der Zahnhöhe, berechnet werden kann und unmittelbar in den Herstellungsprozess einfließen kann.

Durch das Wählen eines geradzahligen Werts für die Anzahl der über den Rohrumfang verteilten Zahnperiodenmuster kann insbesondere im Fall eines einfachen Zahnungsperiodenmusters in einer besonders einfachen Weise eine paarweise punktsymmetrische Anordnung der Zahnperiodenmuster erreicht werden, die eine Fertigung stark vereinfacht und dadurch kostengünstiger macht. Einander gegenüberliegende Zahnperiodenmuster können simultan bearbeitet werden, wobei gleichzeitig ein radialer Verlauf von Schneidkanten gewährleistet werden kann.

Als "Länge" wird hier die Erstreckung des Zahnperiodenmusters bzw. eines einzelnen Zahns in der Umfangsrichtung des Fräser-Schafts bezeichnet, während sich die Bezeichnung "Höhe" auf die axiale Richtung des Fräser-Schafts bezieht.

Statt unmittelbarer Heranziehung des Umfangs des Rohrs, kann der Außendurchmesser des Rohrs als Parameter angegeben werden, wobei dann der Rohrumfang aus dem Außendurchmesser des Fräser-Schafts bestimmt wird. Statt des Außendurchmessers kann beispielsweise auch ein Radius angegeben werden. Der Außendurchmesser bzw. eine zu diesem proportionale Kenngröße kann beispielsweise aus einer Datenbank oder von einer Benutzerschnittstelle eingelesen und vorgegeben werden.

Das Ermitteln einer mittleren gewünschten Zahnhöhe der Fräser-Zahnung kann ebenfalls durch das Einlesen aus einer Datenbank oder von einer Benutzerschnittstelle erfolgen.

Nach einer besonders vorteilhaften Ausgestaltung des erfindungsgemäßen Verfahrens kann nach dem Ermitteln der Zahnperiodenlänge eine Anpassung, insbesondere eine Skalierung der Zahnhöhe oder eine Anpassung der Keilwinkel und/oder Anstellwinkel des Zahnperiodenmusters erfolgen, um die ermittelte Zahnperiodenlänge zur Zahnhöhe in eine vorteilhafte Proportion zu setzen. Dabei können unter Umständen geringe Abweichungen der endgültigen Zahnhöhe von der gewünschten Zahnhöhe wie auch Abweichungen der endgültigen ermittelten Zahnform von der gewünschten Zahnform, wie sie durch die vorgegebenen Kenngrößen des Zahnperiodenmusters bestimmt ist, hingenommen werden.

Eine mögliche Ausgestaltung der oben definierten erfindungsgemäßen Vorrichtung sieht vor, dass die Kenngrößen für das Zahnperiodenmuster wenigstens einen Keilwinkel und/oder einen Anstellwinkel umfassen. Da sowohl die Schnitteigenschaften als auch die Höhen/Längenverhältnisse eines Sägezahns maßgeblich durch diese Winkel bestimmt sind, bilden sie besonders aussagekräftige Kenngrößen.

Wenn das Zahnperiodemuster wenigstens ein Zahnpaar enthält, wobei vorteilhaft ein Bruchteil der Zahnperiodenlänge gebildet wird, um die Länge wenigstens eines Zahns des Zahnperiodenmusters zu bestimmen und/oder wobei insbesondere ein Bruchteil der Zahnhöhe gebildet wird, um die Höhe wenigstens eines Zahns des Zahnperiodenmusters zu bestimmen, kann das erfindungsgemäße Verfahren in einer einfachen Weise von Einzelzähnen auf komplexere Zahnperiodenmuster verallgemeinert werden.

Wenn die Höhe des größeren Zahns und die Höhe des kleineren Zahns ausgehend von einer gemeinsamen Zahnspitzenlinie bestimmt werden, kann eine große Stabilität der größeren Zähne bei hohem Wirkungsgrad der Materialabtragung erreicht werden. Daher eignen sich derartige Knochen-Fräser mit mittelgroßen Doppelzähnen besonders für präzises Arbeiten bei guter Stabilität der Zähne und guter Materialabtragung.

Eine ungünstige Veränderung der Längenverhältnisse des kleineren und der größeren Zahns kann vermieden werden, wenn die Länge wenigstens eines größeren und eines kleineren Zahns des Zahnperiodemusters bestimmt wird, so dass die Proportionen sich nicht durch die Variation der Länge eines einzelnen Zahns verschieben.

Ein für präzises Arbeiten bei guter Stabilität der Zähne und guter Materialabtragung besonders gut bemessener Knochenfräser kann durch das erfindungsgemäße Verfahren erreicht werden, wenn beim Ermitteln der gewünschten Zahnperiodenlänge aus der Zahnhöhe der Fräser-Zahnung die Länge des größeren Zahns des Zahnperiodenmusters derart bestimmt wird, dass sie 2/3 der Zahnhöhe beträgt und wenn die Länge des kleineren Zahns des Zahnperiodenmusters derart bestimmt wird, dass sie 1/3 der Zahnhöhe beträgt, wobei die Zahnperiodenlänge der Summe der Längen der Zähne entspricht.

Um einen Knochenfräser zu erreichen, insbesondere einen solchen mit grober Zahnung, der besonders geeignet für größten Wirkungsgrad bei der Materialabtragung bei guter Stabilität der Zähne ist, wird vorgeschlagen, dass die Höhe des größeren Zahns und die Höhe des kleineren Zahns ausgehend von einer gemeinsamen Zahngrundlinie bestimmt werden. Der Wirkungsgrad bei der Materialabtragung ergibt sich dadurch, dass die kleineren Zähne durch diese Bemaßung unter der Zahnspitzenlinie der größeren Zähne verborgen bleiben.

Es wurde überraschenderweise festgestellt, dass der Wirkungsgrad bei hoher Stabilität dann besonders groß ist, wenn beim Ermitteln der gewünschten Zahnperiodenlänge aus der Zahnhöhe der Fräser-Zahnung die Länge des größeren Zahns des Zahnperiodenmusters derart bestimmt wird, dass sie 3/5 der Zahnhöhe beträgt und dass die Länge des kleineren Zahns des Zahnperiodenmusters derart bestimmt wird, dass sie 2/5 der Zahnhöhe beträgt, wobei die Zahnperiodenlänge der Summe der Längen der Zähne entspricht.

Der Fräser besteht in der Regel aus Edelstahl. Bevorzugt wird die Zahnung dadurch ausgebildet, dass die Zahnung durch mechanische Abtragen oder Abtrennen von Material am distalen Rohrende ausgebildet wird, wobei insbesondere die Zahnung durch Laserschweißen geschaffen wird. Alternativ können auch andere Verfahren wie Schleifen, Fräsen oder auch Ätzen eingesetzt werden.

Weitere Vorteile und Merkmale der Erfindung ergeben sich aus den Ansprüchen und aus der nachfolgenden Beschreibung, in der Ausführungsbeispiele zu der Erfindung im Einzelnen erläutert sind. Dabei zeigen:
- Fig. 1: einen Knochen-Fräser mit einer Fräser-Zahnung;
- Fig. 2: ein Ablaufdiagramm eines Verfahrens zum Bestimmen einer Zahnperiodenlänge einer Fräser-Zahnung;
- Fig. 3: einen Ausschnitt aus einer Fräser-Zahnung zur Illustration von Kenngrößen des Zahnperiodenmusters;
- Fig. 4: ein distales Ende eines Knochen-Fräsers mit einer Fräser-Zahnung von einem CRF-Zahnungstyp;
- Fig. 5: ein distales Ende eines Knochen-Fräsers mit einer Fräser-Zahnung von einem CRM-Zahnungstyp;
- Fig. 6: ein distales Ende eines Knochen-Fräsers mit einer Fräser-Zahnung von einem CRC-Zahnungstyp;
- Fig. 7: ein distales Ende eines Knochen-Fräsers mit einer Fräser-Zahnung von einem CRP-Zahnungstyp; und
- Fig. 8: ein distales Ende eines Knochen-Fräsers mit einer Fräser-Zahnung von einem CRPF-Zahnungstyp.

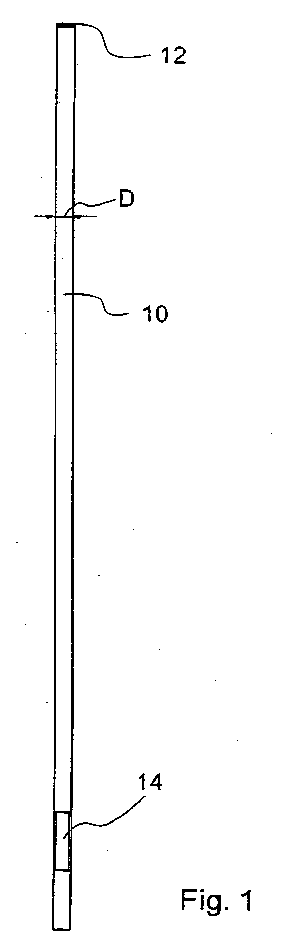

Figur 1 zeigt einen Knochen-Fräser, der einen hohlen zylindrischen Fräser-Schaft 10, an seinem rückwärtigen, proximalen Ende einen hier nicht dargestellten Handgriff und an seinem stirnseitigen bzw. distalen Ende eine Fräser-Zahnung 12 eines ersten Typs aufweist. Der Fräser-Schaft 10 weist an seinem proximalen Ende eine Ausnehmung 14 zum Befestigen des Handgriffs auf.

Ein derartiger Knochen-Fräser wird im Bereich der Medizintechnik zum Ausfräsen von Wirbelbestandteilen im Bereich eines seitlichen Fortsatzes eines Wirbelsäulen-Wirbels eingesetzt, um einen postero-lateralen Zugang zu eingeklemmten Nervenwurzeln des zentralen Nervensystems zu schaffen.

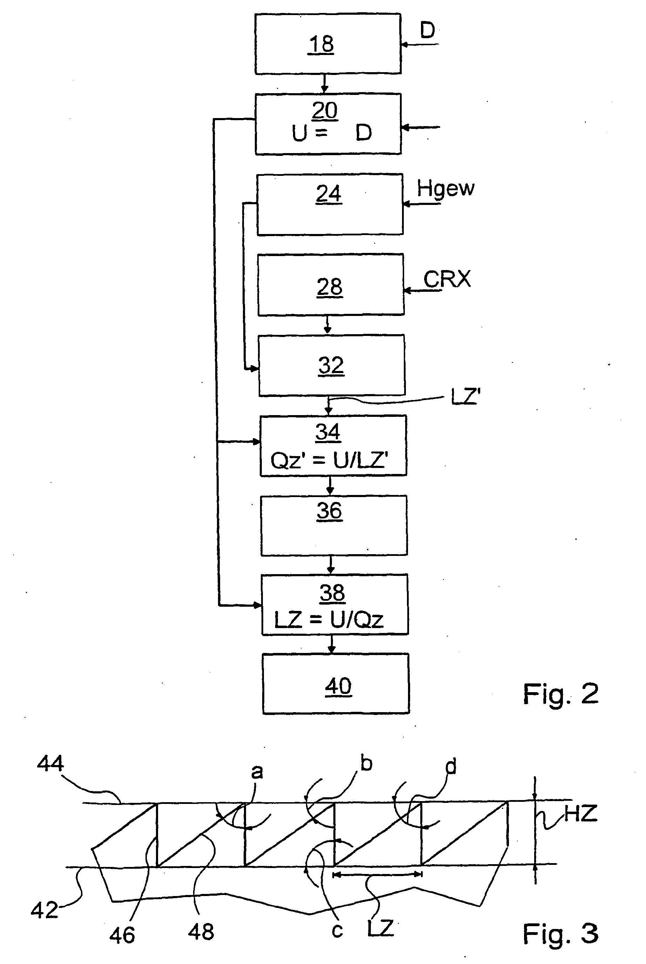

Der in Figur 1 dargestellte Knochen-Fräser ist das unmittelbare Produkt eines Herstellungsverfahrens, welches neben bekannten Bearbeitungsschritten, in welchen bei bekannten Maßen des Knochen-Fräsers dieser aus einem Metallrohr gefertigt wird, ein Verfahren zum Bestimmen der Maße des Knochen-Fräsers umfasst, wie es in der Fig. 2 dargestellt ist. In dem letzteren Verfahren wird insbesondere eine Zahnperiodenlänge LZ einer Fräser-Zahnung 12 an einer distalen Stirnseite eines rohrartigen Fräser-Schafts 10 des Knochen-Fräsers bei vorgegebenem Außendurchmesser D des Fräser-Schafts 10 bestimmt .

Dabei werden insbesondere die in Figur 2 illustrierten Verfahrensschritte durchgeführt.

In einem ersten Schritt 18 wird der Außendurchmesser D von einer hier nicht dargestellten Recheneinheit, die das Verfahren steuert, aus einer Datenbank oder von einer Bedienerschnittstelle eingelesen.

In einem ersten Rechenschritt 20 Berechnet die Recheneinheit den Rohrumfang U aus dem Außendurchmesser D des Fräser-Schafts 10, indem sie den Außendurchmesser D mit der Zahl 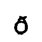 multipliziert, die sie aus einem Festspeicher einliest.

Anschließend ermittelt die Recheneinheit in einem Schritt 24 eine mittlere gewünschte Zahnhöhe Hgew der Fräser-Zahnung 12, indem sie diese von einem Bediener zur Verfügung gestellte Größe einliest.

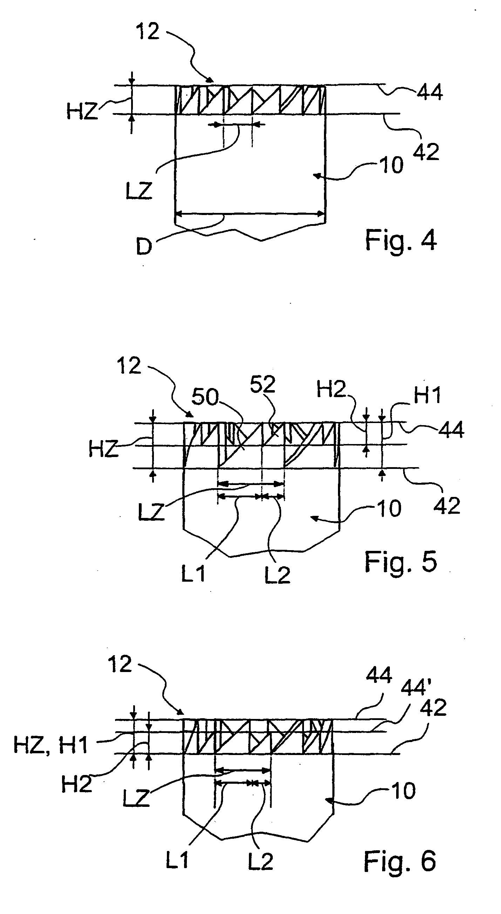

Ferner ermittelt die Recheneinheit in einem Zahnungstypermittlungsschritt 28, mit welchem von 5 verscheiden Zahnungstypen CRX. CRX bezeichnet dabei eine Variable, die den Zahnungstyp codiert, mit welchem der zu konzipierende Knochen-Fräser ausgestattet werden soll. Die Variable CRX kann Werte CRX=CRF, CRM, CRC, CRP oder CRPF annehmen. Die Zahnungstypen CRX werden weiter unten erläutert (Figuren 4 - 8).

Anschließend Ermittelt die Recheneinheit in einem Längenbestimmungsschritt 32 aus der im Schritt ermittelten, gewünschten Zahnhöhe Hgew abhängig von dem durch vorgegebene Kenngrößen eines Zahnperiodenmusters codierten Zahnungstyp CRX einen Sollwert für die Zahnperiodenlänge LZ'.

In einem Divisionsschritt 34 dividiert die Recheneinheit den Rohrumfang U durch die gewünschte Zahnperiodenlänge LZ' bzw. den Sollwert und rundet in einem Rundungsschritt 36 das Ergebnis auf einen geradzahligen Wert Qz.

Schließlich dividiert die Recheneinheit in einem zweiten Divisionsschritt 38 den Rohrumfang U durch den Wert Qz, um die Zahnperiodenlänge LZ zu erhalten.

Nach dem Ermitteln der Zahnperiodenlänge LZ erfolgt in einem Anpassungsschritt 40 eine Skalierung der tatsächlichen Zahnhöhe H durch das Verhältnis aus gewünschter Zahnperiodenlänge LZ' und der im zweiten Divisionsschritt 38 gewonnenen tatsächlichen Zahnperiodenlänge LZ. Durch die Skalierung wird erreicht, dass die Keilwinkel a und/oder Anstellwinkel c des Zahnperiodenmusters unabhängig von der gewünschten Zahnhöhe Hgew sind.

Durch das Wählen eines geradzahligen Werts Qz für die Anzahl der über den Rohrumfang U verteilten Zahnperiodenmuster wird in einer besonders einfachen Weise eine paarweise punktsymmetrische Anordnung der einzelnen Zahnperiodenmuster erreicht, die eine Fertigung stark vereinfacht und dadurch kostengünstiger macht. Einander gegenüberliegende Zahnperiodenmuster können simultan bearbeitet werden, wobei gleichzeitig ein radialer Verlauf von Schneidkanten gewährleistet werden kann.

In vorteilhaften Ausgestaltungen ergeben sich für den Wert Qz gerade Zahlen zwischen 6 und 30 bei Außendurchmessern D zwischen 2 und 9,5mm.

Die Kenngrößen für das Zahnperiodenmuster umfassen beispielsweise einen Keilwinkel a und/oder einen Anstellwinkel c. Daher kann in dem Längenbestimmungsschritt 32 die Zahnlänge bzw. die Zahnperiodenlänge LZ durch einfache trigonometrische Rechnungen bestimmt werden, da die Projektion der Zahnflanken auf die Umfangsrichtung des Fräser-Schafts 10 einfach durch die Multiplikation der gewünschten Zahnhöhe Hgew mit dem Arcus-Cosinus des entsprechenden Winkels berechnet werden kann. Statt der Winkel können natürlich auch direkt der Arcus-Cosinus oder eine andere geeignete trigonometrische Funktion als Kenngröße gespeichert werden.

Die Figuren 3 - 8 zeigen konkrete Ausführungsbeispiele mit verschiedenen Zahnperiodenmustern. Die nachfolgende Beschreibung beschränkt sich auf diejenigen Unterschiede im Verfahren zum Bestimmen der Zahnperiodenlänge LZ, die sich aus den unterschiedlichen Zahnperiodenmustern ergeben, während im Hinblick auf gleich bleibende Merkmale auf die obige allgemeine Beschreibung des Verfahrens verwiesen wird.

Eine erfindungsgemäße Vorrichtung ist zur Durchführung der sogenannten Schritte und zur Schaffung der entsprechenden Zahnung an einem Schaft ausgebildet.

Figur 3 zeigt einen Ausschnitt aus einer Fräser-Zahnung 12 mit einem einfachen Zahnperiodenmuster vom Zahnungstyp 30=CRF mit nur einem Zahn. Der Zahn hat einen Keilwinkel a von 45° und einen Anstellwinkel c von 90°. Das in Figur 4 dargestellte distale Ende eines Knochen-Fräsers ist mit der in Figur 3 dargestellten Fräser-Zahnung 12 ausgestattet. Diese ist besonders zum feinen Arbeiten kurz vor dem Ende der Operation geeignet, wo die Effizienz in der Materialabtragung gegenüber der erforderlichen Präzision in den Hintergrund tritt.

Im Beispiel aus den Figuren 3 und 4 gestaltet sich der Längenbestimmungsschritt 32 besonders einfach, da die Zahnperiodenlänge LZ wegen des Keilwinkel s von 45° gleich der gewünschten Zahnhöhe Hgew ist, so dass die beiden Größen einfach gleich gesetzt werden können. Für den Zahnungstyp CRX=CRF beträgt der Schnittwinkel d=90° und der Freiwinkel b=45°.

Allgemein sind die Kenngrößen des Zahnungsperiodenmusters Winkel zwischen einem Paar von charakteristischen Geraden des Zahnungsperiodenmusters. Die charakteristischen Geraden sind insbesondere die Zahngrundlinie 42, die Zahnspitzenlinie 44, die Zahnbrust 46 und der Zahnrücken 48, wobei im Falle mehrerer Zähne in einem Zahnungsperiodenmuster den Zahnbrüsten 46 und den Zahnrücken 48 unterschiedlicher Zähne auch unterschiedliche Winkel zugeordnet werden können. Den Innenwinkel zwischen Zahnbrust 46 und Zahnrücken 48 bezeichnet man auch als Keilwinkel a, den Innenwinkel zwischen Zahngrundlinie 42 und Zahnbrust 46 als Anstellwinkel c, den Winkel zwischen Zahnrücken 48 und Zahnspitzenlinie 44 als Freiwinkel b und den Winkel zwischen Zahnspitzenlinie 44 und Zahnbrust 46 als Schnittwinkel d.

Wenn das Zahnperiodemuster wenigstens ein Zahnpaar enthält, wie dies bei den in den Figuren 5 und 6 dargestellten Zahnungstypen der Fall ist, bildet die Recheneinheit im Längenbestimmungsschritt 32 einen Bruchteil der gewünschten Zahnhöhe Hgew, um die Höhe und Länge wenigstens eines Zahns des Zahnperiodenmusters zu bestimmen.

In dem in Figur 5 dargestellten Ausführungsbeispiel des Zahnungstyps CRX=CRM wird daher im Längenbestimmungsschritt 32 beim Ermitteln der gewünschten Zahnperiodenlänge LZ aus der gewünschten Zahnhöhe Hgew der Fräser-Zahnung 12 die Länge L1 des größeren Zahns 50 des Zahnperiodenmusters derart bestimmt, dass sie 2/3 der Zahnhöhe Hgew beträgt und die Länge L2 des kleineren Zahns 52 des Zahnperiodenmusters wird derart bestimmt, dass sie 1/3 der Zahnhöhe Hgew beträgt. Der Keilwinkel a der Zähne 50, 52 beträgt 45° und der Anstellwinkel c beträgt 90°. Der Zahnungstyp CRX=CRM ist für präzises Arbeiten bei guter Stabilität der Zähne 50, 52 und guter Materialabtragung konzipiert. Es werden also im Längenbestimmungsschritt 32 sowohl die Länge L2 des kleineren Zahns 52 als auch die Länge L1 des größeren Zahns 50 proportional zur gewünschten Zahnhöhe Hgew bestimmt, so dass die Proportionen unabhängig von der gewünschten Zahnhöhe Hgew festgelegt sind, wobei stets die Zahnperiodenlänge LZ der Summe der Längen L1, L2 der Zähne 50, 52 entspricht.

Die vertikale Anordnung der beiden Zähne 50, 52 des zahnperiodenmusters vom Typ CRM wird so gewählt, dass die Höhe H1 des größeren Zahns 50 und die Höhe H2 des kleineren Zahns 52 ausgehend von einer gemeinsamen Zahnspitzenlinie 44 bestimmt sind. Daher eignen sich derartige Knochen-Fräser mit mittelgroßen Doppelzähnen besonders für präzises Arbeiten bei guter Stabilität der Zähne 50, 52 und guter Materialabtragung.

In vorteilhaften Ausgestaltungen von Knochen-Fräsern mit Zahnperiodenmusters vom Typ CRM ergeben sich für den Wert Qz Zahlen zwischen 2 und 9 bei Außendurchmessern D zwischen 2 und 9,5mm. Die Länge des kleinen Zahns 52 beträgt dann zwischen 1,0472mm und 1,1054mm und die Länge des größeren Zahns 50 beträgt dann zwischen 2,0944mm und 2,2108mm.

Ein für eine hohe Materialabtragung besonders gut bemessener Knochen-Fräser wird durch den Zahnungstyp CRX=CRC erreicht, der in Figur 6 dargestellt ist. Knochen-Fräser mit dem Zahnungstyp CRX=CRC sind besonders geeignet für grobe Vorarbeiten, in denen eine größere Materialmenge abgetragen werden muss und die Präzision nachrangig ist. Beim Ermitteln der gewünschten Zahnperiodenlänge LZ aus der Zahnhöhe Hgew der Fräser-Zahnung 12 wird im Längenbestimmungsschritt 32 Länge L1 des größeren Zahns 50 des Zahnperiodenmusters derart bestimmt, dass sie 3/5 der Zahnhöhe Hgew beträgt und dass die Länge L2 des kleineren Zahns 52 des Zahnperiodenmusters wird derart bestimmt, dass sie 2/5 der Zahnhöhe Hgew beträgt, wobei die Zahnperiodenlänge LZ der Summe der Längen L1, L2 der Zähne 50, 52 entspricht.

In einem Knochen-Fräser vom Zahnungstyp CRX=CRC werden die Höhe H1 des größeren Zahns 50 und die Höhe H2 des kleineren Zahns 52 ausgehend von einer gemeinsamen Zahngrundlinie 42 bestimmt, so dass die Zahnspitzenlinie 44' der kleineren Zähne 52 unterhalb der Zahnspitzenlinie 44 der größeren Zähne 50 liegt. Der Wirkungsgrad bei der Materialabtragung ergibt sich dadurch, dass die kleineren Zähne 52 durch diese Bemaßung unter der Zahnspitzenlinie 44 der größeren Zähne 50 verborgen bleiben.

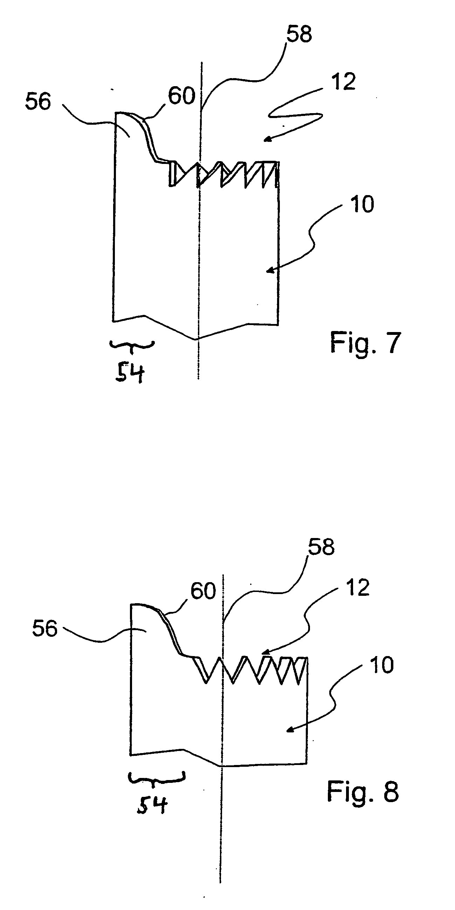

Figur 7 zeigt einen Knochen-Fräser mit einer Fräser-Zahnung 12 vom Zahnungstyp CRX=CRP. Dieser Zahnungstyp ist dadurch gekennzeichnet, dass die Fräser-Zahnung 12 sich nicht über den gesamten Umfang der Stirnseite des Fräser-Schafts 10 erstreckt, sondern in einem Sektor 54 aussetzt, in welchem eine Protektorlippe 56 vorgesehen ist, die die Fräser-Zahnung 12 axial überragt. Die Protektorlippe 56 erstreckt sich über einen Winkelbereich, der prinzipiell frei wählbar ist, üblicherweise jedoch zwischen 90° und 180° liegt, so dass jedenfalls ein Paar von Zahnungsperioden bezüglich der Mittelachse 58 des Fräser-Schafts 10 punktsymmetrisch ist. Die Punktsymmetrie bezieht sich auf die axiale Draufsicht.

Ein derartiger Knochen-Fräser kann eingesetzt werden, um neben dem zu bearbeitenden Knochen angeordnetes Gewebe zu schonen und um Verletzungen zu vermeiden. Im Übrigen ist die Fräser-Zahnung 12 in dem in Figur 6 dargestellten Knochen-Fräser mit der vom Zahnungstyp CRM übereinstimmend.

Figur 8 zeigt einen Knochen-Fräser mit einer Fräser-Zahnung 12 vom Zahnungstyp CRX=CRPF. Dieser Zahnungstyp weist - wie der Zahnungstyp CRX=CRP - eine Protektorlippe mit einer stirnseitigen Raspel 60 auf. Die Zahnung in dem die Protektorlippe zu einem Kreis ergänzenden Bereich der distalen Stirnseite entspricht aber einem Zahnungstyp mit gleichschenkligen Zähnen zum Arbeiten auf Stoß.

Das Ergebnis des oben beschriebenen Herstellungsverfahrens ist unabhängig von der Wahl des Zahnungstyps CRX ein Knochen-Fräser mit einem rohrartigen Fräser-Schaft 10 und einer Fräser-Zahnung 12 an einer distalen Stirnseite des rohrartigen Fräser-Schafts 10, wobei die Fräser-Zahnung 12 von einer Anzahl von periodisch über zumindest einen Teil eines Rohrumfangs U des Fräser-Schafts 10 verteilten Zahnperiodenmustern gebildet ist. Das Charakteristikum des Knochen-Fräsers ist, dass wenigstens ein Paar von Zahnperiodenmustern bezüglich einer Mittelachse 58 des rohrartigen Fräser-Schafts 10 punktsymmetrisch angeordnet ist, weil die Anzahl Qz der Zahnperiodenmuster eine gerade Zahl ist.

Wie auch für den Zahnungstyp CRX=CRF beträgt in den Zahnungstypen CRM, CRC, CRP und CRPF der Schnittwinkel d 90° und der Freiwinkel b 45°. Um das erfindungsgemäße Verfahren auf andere Zahnungstypen zu verallgemeinern, in welchen der Anstellwinkel c von 90° abweicht, können die Zahnlängen im Längenbestimmungsschritt 32 mit Hilfe der Winkelfunktionen einfach aus der gewünschten Zahnhöhe Hgew ermittelt werden. Dadurch können auch Zahnungstypen erreicht werden, mit denen auf Stoß (Anstellwinkel c <90°) oder auf Zug (Anstellwinkel c >90°) gearbeitet werden kann.

Die oben beschriebenen Verfahren sind selbstverständlich auch zur Bemaßung von weiteren, dem Fachmann als sinnvoll erscheinenden Zahnperiodenmustern einsetzbar, beispielsweise für solche mit gekrümmter Zahnbrust oder für solche mit mehr als zwei Zähnen. Die Verfahren sind ferner nicht auf Knochen-Fräser mit rein zylindrischem Fräser-Schaft 10 beschränkt, sondern können vielmehr auch im Zusammenhang mit Fräser-Schäften eingesetzt werden, die sich im Bereich der Fräser-Zahnung 12 oder unterhalb der Fräser-Zahnung 12 radial aufweiten.

### Bezugszeichenliste

- 10: Fräser-Schaft
- 12: Fräser-Zähnung
- 14: Ausnehmung
- 18: Schritt
- 20: Rechenschritt
- 24: Schritt
- 28: Zahnungstypermittlungsschritt
- 32: Längenbestimmungsschritt
- 34: Divisionsschritt
- 36: Rundungsschritt
- 38: Divisionsschritt
- 40: Anpassungsschritt
- 42: Zahngrundlinie
- 44: Zahnspitzenlinie
- 46: Zahnbrust
- 48: Zahnrücken
- 50: Zahn
- 52: Zahn
- 54: Sektor
- 56: Protektorlippe
- 58: Mittelachse
- 60: Raspel
- a: Keilwinkel
- b: Freiwinkel
- c: Anstellwinkel
- d: Schnittwinkel
- L1: Länge
- L2: Länge
- H1: Höhe
- H2: Höhe
- Hgew, HZ: Zahnhöhe
- LZ, LZ': Zahnperiodenlänge
- U: Rohrumfang
- D: Außendurchmesser
- Qz: Wert
- CRX: Zahnungstyp

## Patentansprüche

1. Verfahren zum Herstellen eines Knochen-Fräsers, wobei an einer distalen Stirnseite eines rohrartigen Fräser-Schafts (10) eines Knochen-Fräsers bei vorgegebenem Außendurchmesser (D) des Fräser-Schafts (10) eine Fräser-Zahnung (12) geschaffen wird, wobei die folgenden Schritte zeitlich nacheinander durchgeführt werden:
- Bestimmen des Rohrumfangs (U) des Fräser-Schafts (10);
- Ermitteln einer mittleren gewünschten Zahnhöhe (Hgew) der Fräser-Zahnung (12);
- Ermitteln der gewünschten Zahnperiodenlänge (LZ') der Fräser-Zahnung (12) aus der gewünschten Zahnhöhe (Hgew) bei vorgegebenen Kenngrößen eines Zahnperiodenmusters;
- Dividieren des Rohrumfangs (U) durch die gewünschte Zahnperiodenlänge (LZ');
- Runden des Ergebnisses auf einen ganzzahligen Wert (Qz) :
- Dividieren des Rohrumfangs (U) durch den Wert (Qz), um die Zahnperiodenlänge (LZ) zu erhalten, und abschließend
- mechanisches Abtragen oder Abtrennen von Material am distalen Ende des Fräser-Schafts (10) zur Ausbildung der zuvor bestimmten Fräser-Zahnung (12).

2. Verfahren nach Anspruch 1, **dadurch gekennzeichnet, dass** der Rohrumfang (U) aus dem Außendurchmesser bestimmt wird.

3. Verfahren nach einem der Ansprüche 1 bis 2, **dadurch gekennzeichnet, dass** die Zahnung durch Laserschweißen geschaffen wird.

4. Verfahren nach einem der Ansprüche 1 bis 2, **dadurch gekennzeichnet, dass** die Zahnung durch Schleifen geschaffen wird.

5. Verfahren nach einem der Ansprüche 1 bis 2, **dadurch gekennzeichnet, dass** die Zahnung durch Fräsen geschaffen wird.

6. Verfahren nach einem der Ansprüche 1 bis 2, **dadurch gekennzeichnet, dass** die Zahnung durch Ätzen geschaffen wird.

7. Verfahren nach einem der vorangehenden Ansprüche, **dadurch gekennzeichnet, dass** die Kenngrößen für das Zahnperiodenmuster wenigstens einen Keilwinkel (a) umfassen.

8. Verfahren nach einem der vorhergehenden Ansprüche, **dadurch gekennzeichnet, dass** die Kenngrößen für das Zahnperiodenmuster wenigstens einen Anstellwinkel (c) umfassen.

9. Verfahren nach einem der vorhergehenden Ansprüche, **dadurch gekennzeichnet, dass** das Zahnperiodemuster wenigstens ein Zahnpaar (50, 52) enthält.

10. Verfahren nach Anspruch 9, **dadurch gekennzeichnet, dass** ein Bruchteil der Zahnperiodenlänge (LZ) gebildet wird, um die Länge (L1, L2) wenigstens eines Zahns (50, 52) des Zahnperiodenmusters zu bestimmen.

11. Verfahren nach Anspruch 9 oder 10, **dadurch gekennzeichnet, dass** ein Bruchteil der Zahnhöhe (Hgew) gebildet wird, um die Höhe (H1, H2) wenigstens eines Zahns (50, 52) des Zahnperiodenmusters zu bestimmen.

12. Verfahren nach einem der Ansprüche 9 bis 11, **dadurch gekennzeichnet, dass** die Höhe (H1, H2) wenigstens eines größeren und eines kleineren Zahns (50, 52) des Zahnperiodemusters bestimmt wird.

13. Verfahren nach einem Anspruch 12, **dadurch gekennzeichnet, dass** die Höhe (H1) des größeren Zahns (50) und die Höhe (H2) des kleineren Zahns (52) ausgehend von einer gemeinsamen Zahnspitzenlinie (44) bestimmt werden.

14. Verfahren nach einem der Ansprüche 9 bis 13, **dadurch gekennzeichnet, dass** die Länge (L1, L2) wenigstens eines größeren und eines kleineren Zahns (50, 52) des Zahnperiodemusters bestimmt wird.

15. Verfahren nach Anspruch 14, **dadurch gekennzeichnet, dass** beim Ermitteln der gewünschten Zahnperiodenlänge (LZ') aus der gewünschten Zahnhöhe (Hgew) der Fräser-Zahnung (12) die Länge (L1) des größeren Zahns (50) des Zahnperiodenmusters derart bestimmt wird, dass sie 2/3 der Zahnhöhe (Hgew) beträgt und dass die Länge (L2) des kleineren Zahns (52) des Zahnperiodenmusters derart bestimmt wird, dass sie 1/3 der Zahnhöhe (Hgew) beträgt, wobei die Zahnperiodenlänge (LZ) der Summe der Längen (L1, L2) der Zähne (50, 52) entspricht.

16. Verfahren nach einem Anspruch 12, **dadurch gekennzeichnet, dass** die Höhe (H1) des größeren Zahns (50) und die Höhe (H2) des kleineren Zahns (52) ausgehend von einer gemeinsamen Zahngrundlinie (42) bestimmt werden.

17. Verfahren nach einem der Ansprüche 12 bis 14 oder 16, **dadurch gekennzeichnet, dass** beim Ermitteln der gewünschten Zahnperiodenlänge (LZ) aus der Zahnhöhe (Hgew) der Fräser-Zahnung (12) die Länge (L1) des größeren Zahns (50) des Zahnperiodenmusters derart bestimmt wird, dass sie 3/5 der gewünschten Zahnhöhe (Hgew) beträgt und dass die Länge (L2) des kleineren Zahns (52) des Zahnperiodenmusters derart bestimmt wird, dass sie 2/5 der Zahnhöhe (Hgew) beträgt, wobei die Zahnperiodenlänge (LZ) der Summe der Längen (L1, L2) der Zähne (50, 52) entspricht.

18. Vorrichtung zur Ausführung eines Verfahrens zum Herstellen von Knochen-Fräsern nach einem der Ansprüche 1-17, mit der an einer distalen Stirnseite eines rohrartigen Fräser-Schafts (10) eines Knochen-Fräsers bei vorgegebenem Außendurchmesser (D) des Fräser-Schafts (10) eine Fräser-Zahnung (12) geschaffen wird, **dadurch gekennzeichnet, dass** die Vorrichtung mit einer Recheneinheit und einer Abtragungs- oder Abtrennungsvorrichtung zur Durchführung der Verfahrensschritte ausgebildet ist.

19. Vorrichtung nach Anspruch 18, **dadurch gekennzeichnet, dass** die Kenngrößen für das Zahnperiodenmuster wenigstens einen Keilwinkel und/oder einen Anstellwinkel umfassen.

## Claims

1. A method for manufacturing a bone milling cutter, wherein a milling cutter toothing (12) is created at a distal face of a tubular milling cutter shaft (10) of a bone milling cutter and at a given outer diameter (D) of the milling cutter shaft (10), **characterized by** the following steps, executed in sequence:
determining the tube circumference (U) of the milling cutter shaft (10);
ascertaining an average desired tooth height (Hgew) of the milling cutter toothing (12);
ascertaining the desired tooth period length (LZ') of the milling cutter toothing (12) from the desired tooth height (Hgew) at given parameters of a tooth period pattern;
dividing the tube circumference (U) by the desired tooth period length (LZ);
rounding the results to an even number value (Qz);
dividing the tube circumference (U) by the value (Qz) in order to obtain the tooth period length (LZ); and finally mechanically removing or severing material at the distal end of the milling cutter shaft (10) to configure the previously determined milling cutter toothing (12).

2. The method according to claim 1, **characterized in that** the tube circumference (U) is determined by the outer diameter.

3. The method according to claim 1 or 2, **characterized in that** the toothing is created by means of laser welding.

4. The method according to claim 1 or 2, **characterized in that** the toothing is created by grinding.

5. The method according to claim 1, **characterized in that** the toothing is created by milling.

6. The method according to claim 1 or 2, **characterized in that** the toothing is created by etching.

7. The method according to any one of the previous claims, **characterized in that** the parameters for the tooth period pattern comprise at least one wedge angle (a).

8. The method according to any one of the previous claims, **characterized in that** the parameters for the tooth period pattern comprise at least one blade angle (c).

9. The method according to any one of the previous claims, **characterized in that** the tooth period pattern contains at least one pair of teeth (50, 52).

10. The method according to claim 9, **characterized in that** a fraction of the tooth period length (LZ) is formed in order to determine the length (L1, L2) of at least one tooth (50, 52) of the tooth period pattern.

11. The method according to claim 9 or 10, **characterized in that** a fraction of the tooth height (Hgew) is formed in order to determine the height (H1, H2) of at least one tooth (50, 52) of the tooth period pattern.

12. The method according to any one of claims 9 to 11, **characterized in that** the height (H1, H2) of at least one larger and one smaller tooth (50, 52) of the tooth period pattern is determined.

13. The method according to claim 12, **characterized in that** the height (H1) of the larger tooth (50) and the height (H2) of the smaller tooth (52) are determined starting from a common tooth tip line (44).

14. The method according to any one of claims 9 to 13, **characterized in that** the length (L1, L2) of at least one larger and one smaller tooth (50, 52) of the tooth period pattern is determined.

15. The method according to claim 14, **characterized in that**, when ascertaining the desired tooth period length (LZ') from the desired tooth height (Hgew) of the milling cutter toothing (12), the length (L1) of the larger tooth (50) of the tooth period pattern is determined such that it is 2/3 of the tooth height (Hgew) and **in that** the length (L2) of the smaller tooth (52) of the tooth period pattern is determined such that it is 1/3 of the tooth height (Hgew), wherein the tooth period length (LZ) corresponds to the sum of the lengths (L1, L2) of the teeth (50, 52).

16. The method according to one claim 12, **characterized in that** the height (H1) of the larger tooth (50, 52) and the height (H2) of the smaller tooth (52) is determined starting from a common tooth base line (42).

17. The method according to any one of claims 12 to 14 or 16, **characterized in that**, when ascertaining the desired tooth period length (LZ) from the tooth height (Hgew) of the milling cutter toothing (12), the length (L1) of the larger tooth (50) of the tooth period pattern is determined such that it is 3/5 of the desired tooth height (Hgew) and the length (L2) of the smaller tooth (52) of the tooth period pattern is determined such that it is 2/3 of the tooth height (Hgew), wherein the tooth period length (LZ) corresponds to the sum of the lengths (L1, L2) of the teeth (50, 52).

18. A device to execute a method for manufacturing bone milling cutters according to any one of the claims 1 - 17, with which a milling cutter toothing (12) is created at a distal face of a tubular milling cutter shaft (10) of a tubular milling cutter at a given outer diameter (D) of the milling cutter shaft (10), **characterized in that** the device is configured for carrying out the method steps with an arithmetic unit and an abrasive or abscission device.

19. The device according to claim 18, **characterized in that** the parameters for the tooth period pattern comprise at least one wedge angle and/or one blade angle.

## Revendications

1. Procédé de fabrication d'une fraise à os, une denture de fraise (12) étant créée au niveau d'une face frontale distale d'une tige de fraise (10) de type tubulaire d'une fraise à os avec un diamètre extérieur (D) prédéfini de la tige de fraise (10), les étapes suivantes étant réalisées les unes après les autres dans le temps :
- détermination du périmètre de tube (U) de la tige de fraise (10) ;
- calcul d'une hauteur de dent moyenne souhaitée (Hgew) de la denture de fraise (12) ;
- calcul de la longueur périodique de dent souhaitée (LZ') de la denture de fraise (12) à partir de la hauteur de dent souhaitée (Hgew) avec des grandeurs caractéristiques prévues pour un modèle de période de dent ;
- division du périmètre de tube (U) par la longueur périodique de dent souhaitée (LZ') ;
- arrondi du résultat à une valeur entière (Qz) ;
- division du périmètre de tube (U) par la valeur (Qz), pour obtenir la longueur périodique de dent (LZ) ; puis
- démontage ou séparation mécanique de matériau au niveau de l'extrémité distale de la tige de fraise (10) pour former la denture de fraise (12) préalablement définie.

2. Procédé selon la revendication 1, **caractérisé en ce que** le périmètre de tube (U) est déterminé à partir du diamètre extérieur.

3. Procédé selon l'une quelconque des revendications 1 à 2, **caractérisé en ce que** la denture est créée par soudage laser.

4. Procédé selon l'une quelconque des revendications 1 à 2, **caractérisé en ce que** la denture est créée par meulage.

5. Procédé selon l'une quelconque des revendications 1 à 2, **caractérisé en ce que** la denture est créée par fraisage.

6. Procédé selon l'une quelconque des revendications 1 à 2, **caractérisé en ce que** la denture est créée par gravure.

7. Procédé selon l'une quelconque des revendications précédentes, **caractérisé en ce que** les grandeurs caractéristiques du modèle de période de dent comprennent au moins un angle de cale (a).

8. Procédé selon l'une quelconque des revendications précédentes, **caractérisé en ce que** les grandeurs caractéristiques du modèle de période de dent comprennent au moins un angle d'appui (c).

9. Procédé selon l'une quelconque des revendications précédentes, **caractérisé en ce que** le modèle de période de dent comprend au moins une paire de dents (50, 52).

10. Procédé selon la revendication 9, **caractérisé en ce qu'**une pièce de rupture de la longueur périodique de dent (LZ) est formée pour déterminer la longueur (L1, L2) d'au moins une dent (50, 52) du modèle de période de dent.

11. Procédé selon la revendication 9 ou 10, **caractérisé en ce qu'**une pièce de rupture de la hauteur de dent (Hgew) est formée, pour déterminer la hauteur (H1, H2) d'au moins une dent (50, 52) du modèle de période de dent.

12. Procédé selon l'une quelconque des revendications 9 à 11, **caractérisé en ce que** la hauteur (H1, H2) d'au moins une dent relativement grande et d'une dent relativement petite (50, 52) du modèle de période de dent est déterminée.

13. Procédé selon la revendication 12, **caractérisé en ce que** la hauteur (H1) de la dent relativement grande (50) et la hauteur (H2) de la dent relativement petite (52) sont déterminées à partir d'une ligne de sommets de dent commune (44).

14. Procédé selon l'une quelconque des revendications 9 à 13, **caractérisé en ce que** la longueur (L1, L2) d'au moins une dent relativement grande et une dent relativement petite (50, 52) du modèle de période de dent est déterminée.

15. Procédé selon la revendication 14, **caractérisé en ce que** lors du calcul de la longueur périodique de dent souhaitée (LZ'), la longueur (L1) de la dent relativement grande (50) du modèle de période de dent est déterminée de telle sorte à partir de la hauteur de dent souhaitée (Hgew) de la denture de fraise (12) qu'elle représente 2/3 de la hauteur de dent (Hgew) et que la longueur (L2) de la dent relativement petite (52) du modèle de période de dent est déterminée de telle sorte qu'elle représente 1/3 de la hauteur de dent (Hgew), la longueur périodique de dent (LZ) correspondant à la somme des longueurs (L1, L2) des dents (50, 52).

16. Procédé selon la revendication 12, **caractérisé en ce que** la hauteur (H1) de la dent relativement grande (50) et la hauteur (H2) de la dent relativement petite (52) sont déterminées à partir d'une ligne de base de dent commune (42).

17. Procédé selon l'une quelconque des revendications 12 à 14 ou 16, **caractérisé en ce que** lors du calcul de la longueur périodique de dent souhaitée (LZ) à partir de la hauteur de dent (Hgew) de la denture de fraise (12), la longueur (L1) de la dent relativement grande (50) du modèle de période de dent est déterminée de façon à représenter 3/5 de la hauteur de dent souhaitée (Hgew) et que la longueur (L2) de la dent relativement petite (52) du modèle de période de dent est déterminée de façon à représenter 2/5 de la hauteur de dent (Hgew), la longueur périodique de dent (LZ) correspondant à la somme des longueurs (L1, L2) des dents (50, 52).

18. Dispositif de réalisation d'un procédé de fabrication d'une fraise à os selon l'une quelconque des revendications 1 à 17, avec lequel une denture de fraise (12) est créée au niveau d'une face frontale distale d'une tige de fraise (10) de type tubulaire d'une fraise à os avec un diamètre extérieur prédéfini (D) de la tige de fraise (10), **caractérisé en ce que** le dispositif est réalisé avec une unité de calcul et un dispositif de démontage ou de séparation permettant de réaliser les étapes de procédé.

19. Dispositif selon la revendication 18, **caractérisé en ce que** les grandeurs caractéristiques du modèle de période de dent comprennent au moins un angle de cale et/ou un angle de réglage.
